# EUROPEAN PATENT APPLICATION

(11) **EP 1 302 208 A1**
(43) Date of publication of application: **16.04.2003**
(21) Application number: 01830650.6
(22) Date of filing: 16.10.2001
(51) Int. Cl.: A61L 2/20, B08B 9/02, C02F 1/78

(54) **Apparatus and process for drying and sterilising industrial plants**

(71) Applicant: Ico Oleodinamici S.p.A., 41010 Frazione San Damaso (MO) (IT)
(72) Inventor: Barani, Ruggero, 41100 Modena (IT)
(74) Representative: Gotra, Stefano

(57) **Abstract**

The drying and sterilising apparatus comprises an ozoniser (10) which takes oxygen from a tank (11), transforming it into ozone which is then conveyed to a drying line (16) which takes drying air from the atmosphere and sends it to the surfaces of the plant to be treated. The ozone mixes with the drying air, forming a mix which strikes the surfaces of the plant, drying and sterilising them. A second automatic sterilising line (21) is predisposed to cause the mix of ozone and air to flow along the drying line in a closed circuit. The invention is particularly applicable to plants used in the pharmaceutical industry, such as for example granulation plants. The plant dries and sterilises the treated surfaces with remarkable efficiency and economy.

## Description

Especially, though not exclusively, the invention is usefully applied in plants in the pharmaceutical industry, such as for example granulation, mixing, dissolving, coating solid particles, bins, tanks etc.

The main aim of the present invention is to provide an especially efficient process for automatic drying of the treated surfaces (especially after washing) of an industrial plant.

An advantage of the invention is that it profoundly sterilises the surfaces of the plant subjected to the drying treatment.

A further aim is to provide a simple and economical apparatus for realising the above-cited process.

These aims and advantages and others besides are all attained by the invention as it is characterised in the appended claims.

Further characteristics and advantages of the present invention will better emerge from the detailed description that follows of a preferred but non-exclusive embodiment of the invention, illustrated purely by way of a nonlimiting example in the accompanying figure of the drawing, in which:

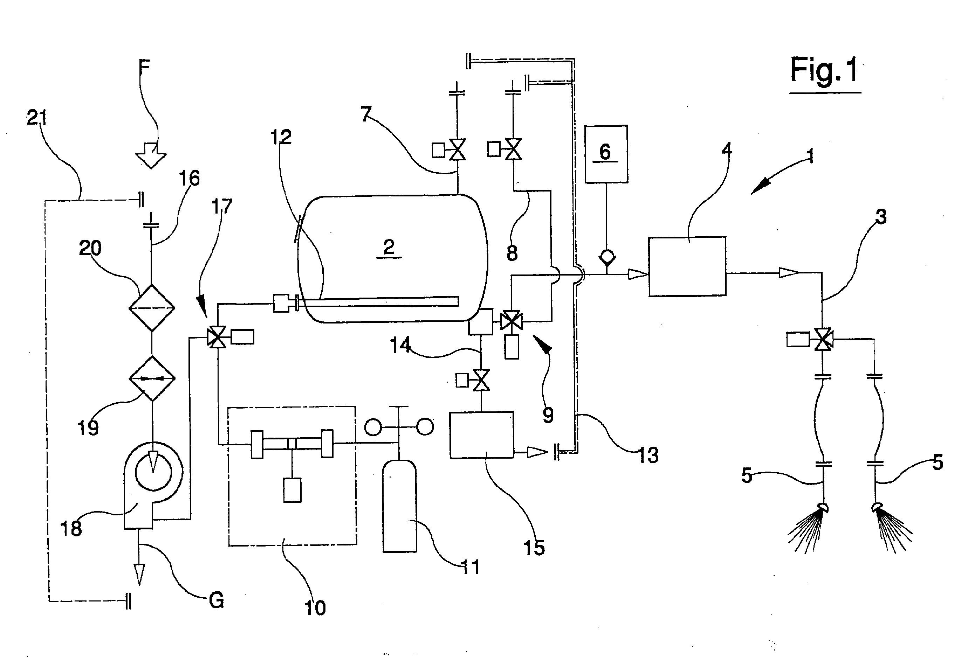

figure 1 is a diagram of an apparatus realised according to the invention.

With reference to the figure of the drawing, 1 denotes in its entirety a plant comprising two combined apparatus which cooperate with one another. A first apparatus is for drying and sterilising industrial plants and a second apparatus is for washing industrial plants.

The drying apparatus comprises a drying line 16 predisposed to supply a drying gas to the treated surfaces of the industrial plant (of known type and not illustrated).

The drying line 16 is provided with a feed line which removes air from the atmosphere (in the direction indicated by arrow F); along the feed line means for moving the air are arranged (for example an aspirating fan 18), at least one heater 19 and at least one filter 20. The drying gas is sent towards the plant to be dried in the direction of arrow G.

The drying line 16 is connected through a conduit and a valve 17 to a gas feed line on which an ozonizer 10 (provided with a voltage generator) is provided. The ozoniser 10 takes oxygen from a tank 11 and transforms it into ozone. Any fixed supply line of oxygen can be used. In the illustrated embodiment the valve is a three-way switch valve 17 to the feed line which introduces the sterilising gas into at least one user (in this embodiment the second washing apparatus, as will be better explained herein below), as well as enabling the sterilising gas - ozone - to be mixed with the drying gas - air before the mixture is sent on to the surfaces to be treated. Predisposing at least one further user for use with the sterilising gas supply is optional, preferable but not necessary.

The drying line 16 is associated to a secondary recycling line 21 which is selectively connectable to the drying line 16 (when the latter is operative on the industrial plant), to create a closed automatic sterilising circuit of the drying line 16. The secondary recycling line 21 is basically a by-pass for performing, when so desired, an automatic sterilisation of the drying line 16.

The above-described first drying apparatus can be used alone for independently drying, or can be advantageously combined with the washing apparatus in order to wash the surfaces which are destined to be subsequently dried by the drying apparatus.

There follows a description of the preferable though optional part of the plant 1 which is destined to the task of washing the industrial plant, which is thereafter dried by means of the drying line 16. The washing apparatus comprises a tank 2 containing a washing liquid (for example, water) connected, through a high-pressure feed line 3 comprising a pipe including a pump 4 (for example a 70-bar pressure pump), to one or more washing terminals 5 (for example water nozzles). The washing terminals 5 are associated with the surfaces of the plant which are to be treated. In the illustrated embodiment there is also a batcher 6 which feeds the line 3 with measured amounts of a detergent at a non-pressurised point upstream of the pump 4.

A short line 7 is attached to the tank 2, which will removably join up with an external line supplying the washing fluid, for example the municipal water supply. A further short line 8, also removably connectable to the water supply, joins the high-pressure feed line 3 at a three-way switch valve 9 predisposed (upstream of the high-pressure pump and at a non-pressurised point) selectively to connect the high-pressure feed line 3 with the tank supply 2 or with the municipal water supply through the connecting line 8. Both short connecting lines 7 and 8 are provided with an intercept valve.

The washing apparatus 1 comprises means for introducing a sterilising gas into the tank 2, which gas mixes with the washing liquid. The means for introducing the sterilising gas comprise a gas feed line which, through a switch valve 17, can selectively send the gas to the drying line 16 or the tank 2. The oxygen supply line terminates inside the tank 2 with an injector 12 located close to the bottom of the tank 2 which releases the sterilising gas (ozone) into the washing liquid, thus obtaining a mixture of the two substances.

The apparatus comprises a secondary automatic sterilising line 13 predisposed to connect selectively the tank 2 with parts of the apparatus which during normal functioning are not crossed by the washing liquid, so that these parts too can be sterilised. In the illustrated embodiment these non-contact parts are the two short water lines 7 and 8 which supply the washing liquid (water) to the tank 2, and the high-pressure feed line 3. During automatic sterilisation the ends of the short lines 7 and 8 are detached from the municipal water supply and joined up to the secondary automatic sterilising line 13. The secondary line 13 exhibits at one end a removable and selective connection with the short lines 7 and 8; while at its other end it exhibits a connector for selective and removable connection with a subsidiary draining line 14 of the tank 2, provided with a draining pump 15, which can on demand be used both for draining liquid remaining in the tank 2 and for automatic sterilisation of parts of the apparatus.

The various above-mentioned parts of the apparatus 1 - in particular the tank 2, the high-pressure feed line 3, the pump 4, the washing terminals 5 and the means for introducing sterilising gas, but also the drying system as well as the auto-sterilising systems with ozonised water and ozonised air - are mounted on a mobile washing unit with wheels (not illustrated).

The apparatus enables automatic sterilisation and cleaning of structural components (the drying line 16 and the washing system, if present) with no need to dismount them: this is realised by means of the on-site production of sterilising gas (ozone) which is mixed with the drying gas (or washing liquid) and thus conveyed at low temperature to the inside of the hydraulic circuit of the apparatus. This solution means that the same control unit can be programmed to command both the washing/drying operations and the automatic sterilising cycle.

Cold automatic sterilising (instead of, for example, sterilising using heated steam) means that relatively-cheap parts of the apparatus itself can be used in the operation, as no compatibility with high temperatures is required.

Production and introduction of sterilising gas into the tank 2 are controlled (using known techniques) so that the saturation level of sterilising gas (in this embodiment ozone) mixed with the washing liquid (in this case water) in the tank 2 is never exceeded, so no over-production of ozone obtains.

It has been seen that by using a high-pressure washing liquid and a mixed-in sterilising gas (i.e. in the present embodiment ozonised water) an extremely efficient wash and sterilisation is obtained of all of the treated surfaces of the plant. In the case of ozonised water, high-pressure delivery over the surfaces to be treated accelerates the transformation of the ozone contained in the water into active oxygen, which is able to sterilise even those parts of the machine which are not directly sprayed by the jets of pressurised liquids.

## Claims

1. An apparatus for drying and sterilising industrial plants, comprising a drying line (16) for feeding a drying gas to a surface to be treated of a plant, and means (10, 11) for introducing a sterilising gas to the drying line which sterilising gas mixes with the drying gas.

2. The apparatus of claim 1, **characterised in that** the means for introducing the sterilising gas comprise an ozoniser (10) which removes oxygen from a tank (11) and transforms the oxygen into ozone, which is conveyed to the drying line (16).

3. The apparatus of claim 1 or 2, comprising a secondary line (21) for recycling, selectively connected to the drying line in order to realise a sterilising closed circuit of the drying line (16).

4. The apparatus of any one of the preceding claims, **characterised in that** the apparatus is combined with a washing apparatus comprising at least one tank (2) containing a washing liquid connected, through at least one high-pressure feed line (3) provided with at least one pump (4), at least one washing terminal (5) operatively associated to a surface to be treated; also comprising means (10, 11, 12) for introducing a sterilising gas into the tank (2) which mixes with the washing liquid, the means for introducing being wholly or partially constituted by the means for introducing the sterilising gas into the drying line, or being different from the means for introducing.

5. The apparatus of claim 4, **characterised in that** it comprises at least one secondary line for automatic sterilisation (13) which can selectively place in a closed-circuit communication the tank (2) with parts of the apparatus which during normal functioning are not crossed by the washing liquid, in order that the parts of the apparatus which during normal functioning are not crossed by the washing liquid can be washed.

6. The apparatus of any one of the preceding claims, **characterised in that** at least the tank (2), the high-pressure feed line (3), the pump (4), the washing terminal (5) and the means (10, 11, 12) for introducing the sterilising gas are mounted on a compact washing unit which is self-sufficient and mobile on wheels.

7. A process for drying and sterilising industrial plants wherein a drying gas liquid is sourced from a source which can be a surrounding normal atmosphere and is sent through one or more pipes towards at least one surface of a plant to be dried and sterilised, which plant is sprayed by the drying gas, **characterised in that** a sterilising gas is introduced internally of the pipes, which sterilising gas mixes with the drying gas.

8. The process of claim 7, **characterised in that** the sterilising gas comprises ozone.

9. The process of claim 7 or 8, **characterised in that** the process is realised by an apparatus according to any of claims from 1 to 5 and **characterised in that** a part of the apparatus is automatically sterilised by the closed-circuit drying flow of drying gas.
